Europäisches Patentamt

European Patent Office

Office européen des brevets

(19)

(11) **EP 0 722 299 B1**

(12) **FASCICULE DE BREVET EUROPEEN**

(45) Date de publication et mention
de la délivrance du brevet:
**12.01.2000 Bulletin 2000/02**

(21) Numéro de dépôt: **94926960.9**

(22) Date de dépôt: **06.09.1994**

(51) Int. Cl.7: **A61B 19/00**

(86) Numéro de dépôt international:
**PCT/FR94/01050**

(87) Numéro de publication internationale:
**WO 95/07055 (16.03.1995 Gazette 1995/12)**

(54) **INSTALLATION POUR OPERATION DE MICROCHIRURGIE ASSISTEE PAR ORDINATEUR ET
PROCEDES MIS EN ŒUVRE PAR LADITE INSTALLATION**

COMPUTERUNTERSTÜTZTE MIKROCHIRURGIEAUSRÜSTUNG SOWIE DIESE AUSRÜSTUNG
GEBRAUCHENDE VERFAHREN

COMPUTER-ASSISTED MICROSURGERY EQUIPMENT AND METHODS FOR USE WITH SAID
EQUIPMENT

(84) Etats contractants désignés:
**AT BE CH DE DK ES FR GB GR IE IT LI LU MC NL
PT SE**

(30) Priorité: **07.09.1993 FR 9310624**

(43) Date de publication de la demande:
**24.07.1996 Bulletin 1996/30**

(73) Titulaire: **ELEKTA IGS S.A.
38610 Gières (FR)**

(72) Inventeur: **DRUAIS, Hervé
F-38000 Grenoble (FR)**

(74) Mandataire: **Pernez, Helga
Breese-Majerowicz,
3, avenue de l'Opéra
75001 Paris (FR)**

(56) Documents cités:
**WO-A-91/04711**

**Description**

La présente invention concerne une installation pour opération de microchirurgie stéréotaxique assistée par ordinateur.

ETAT DE LA TECHNIQUE

On connaît dans l'état de la technique de telles installations. A titre d'exemple, le brevet français FR2651670 décrit un procédé de localisation précise d'une lésion et dispositif pour la mise en oeuvre de ce procédé.

L'invention concerne un dispositif et un procédé de localisation précise d'une lésion. Le procédé selon l'invention est caractérisé en ce qu'on immobilise l'organe à examiner dans la même position que celle de la biopsie à pratiquer, on effectue des coupes axiales (XY) tomodensitomètriques de l'organe à travers au moins un rectangle transparent muni de trois fils opaques concourants non symétriques occupant des positions déterminées par rapport à l'appareillage de biopsie, on mesure les longueurs des deux segments (AB, AC) interceptés par lesdits fils opaques pour une coupe lésionnelle choisie, on effectue au moins un cliché en position de prélèvement, on reconstruit sur ledit cliché la trace des trois fils opaques, et on reporte sur ledit cliché les longueurs des segments (AB, AC) mesurés pour déterminer la ligne de base lésionnelle correspondant à la coupe lésionnelle choisie.

La mise en oeuvre de ce procédé implique une immobilisation parfaite du patient.

Un autre brevet français publié sous le numéro FR2686499 décrit un appareil de traitement d'une cible, telle qu'une lésion à l'intérieur du corps utilisant un élément marqueur implanté dans ou au voisinage de la cible pour piloter la thérapie de ladite cible. Cet appareil de thérapie comprend:

- des moyens de thérapie de la lésion,
- des moyens de repérage de la lésion, les moyens de repérage étant liés, par exemple mécaniquement ou électriquement, aux moyens de thérapie
- des moyens de calcul de la position de la lésion relativement aux moyens de thérapie à l'aide des moyens de repérage
- des moyens d'activation des moyens de thérapie.

Les moyens de repérage réalisent le repérage d'au moins un élément marqueur implanté à l'intérieur de la lésion. Les moyens de calcul calculent les coordonnées de position de l'élément marqueur (M0, M1, M2, M3) par rapport aux moyens de thérapie qui sont utilisés pour positionner les moyens de thérapie mobiles dans l'espace dans une position quelconque selon les axes X, Y, Z. Cet appareil permet de réaliser une thérapie précise de la lésion.

Un tel appareil nécessite, pour sa mise en oeuvre, une préparation pré-chirurgicale lourde.

Le brevet français FR2682778 décrit un microscope pour opération de microchirurgie stéréotaxique assistée par ordinateur, et un procédé pour son fonctionnement. Ce microscope comporte des détecteurs détectant des données optiques, un système d'identification de position et un dispositif de commande de processus évaluant les signaux dudit système. Ce système est un système à base optique intégré dans le système optique du microscope et il est prévu un dispositif qui convertit les signaux délivrés par le dispositif en une représentation graphique bidimensionnelle.

Un autre brevet de l'art antérieur, le brevet PCT/FR090/00714, divulgue une installation dans laquelle le référentiel principal est lié au lit du patient. Le patient est immobilisé par rapport au lit par un casque de maintien ou un moyen équivalent. Ce document de l'art antérieur expose que le système comporte des moyens notés 2, de mise en position liée, par rapport au référentiel R2 des structures SNH et SR. A titre d'exemple, la tête est fixée sur une table d'opération.

Cette solution n'est pas totalement satisfaisante car les moyens de maintien réduisent les voies d'accès possibles, et imposent des contraintes gênantes au chirurgien, qui doit considérer que la position du patient est définitive à partir du début de l'intervention.

De plus, le lit opératoire ne présente jamais une rigidité mécanique absolue, et la corrélation entre le patient et les images virtuelles ne présente pas un degré de précision suffisant pour certaines interventions.

Le brevet WO92/06644 décrit une installation de radiothérapie comportant des moyens de mise en concordance des sources de rayonnement et des images obtenues préalablement. Ce document ne mentionne pas la mise en oeuvre d'un référentiel correspondant au référentiel fixe de l'invention de la demanderesse, qui n'est d'ailleurs pas nécessaire compte tenu des applications envisagées dans ce document de l'art antérieur.

OBJET DE LA PRESENTE INVENTION

L'objet de la présente invention est de remédier à ces inconvénients en proposant une installation d'utilisation ergonomique, permettant de dissocier la phase d'acquisition d'image et la phase d'exploitation des images à des fins chirurgicales.

Dans l'état de la technique, les systèmes d'acquisition d'images à des fins de diagnostiques, ne nécessitant pas une intervention lourde ou traumatisante, ne sont pas exploitables à des fins per-opératoires. En effet, l'imagerie per-opératoires nécessite le recours à des techniques de stéréotaxie contraignantes pour le patient et pour le personnel opératoire. Ces techniques comportent notamment une phase douloureuse d'implantation d'une structure mécanique en forme de cadre qui est indispensable afin d'acquérir les images par rapport à un référentiel fixe connu, de permettre une calibration satisfaisante des images, et d'assurer l'immobilisation de la tête du patient, ou plus généralement de la zone opératoire, par rapport à un référentiel donné.

Le but de l'invention est d'assurer une corrélation entre des images numériques obtenues par un système d'imagerie médicale avec le patient de façon à apporter au chirurgien des informations destinées à guider en temps réelle sa stratégie opératoire. Certaines interventions nécessitent une précision de la corrélation de l'ordre du millimètre, voire inférieure au millimètre.

Pour atteindre ce but, l'installation selon l'invention comporte un référentiel absolu qui est le référentiel fixe Rr lié à une structure totalement indépendante du patient ou du système d'imagerie ou de visualisation.

Un autre but de l'invention est de permettre aux chirurgiens de procéder à l'acquisition des images sur un patient non anesthésié et autonome, suivant une procédure simplifiée, à n'importe quel moment de l'hospitalisation, voire dans un établissement hospitalier différent, et éventuellement recourir à plusieurs techniques d'imagerie complémentaires.

L'invention concerne plus particulièrement une installation du type comportant un support d'outils articulé dont l'une des extrémités est solidaire d'un référentiel fixe $R_c$, ledit système comportant des moyens pour déterminer les coordonnées (position d'un point et orientation d'un vecteur directeur) de l'outil dans ledit référentiel fixe $R_c$, ainsi qu'une base de données Images dans laquelle sont enregistrées les images provenant d'un système d'imagerie dans le référentiel de l'image $R_i$. L'installation selon l'invention comporte au moins deux capteurs solidaires du référentiel fixe $R_c$ délivrant un signal électrique fonction de la position du référentiel du patient $R_p$ dans le référentiel fixe $R_c$, et un calculateur pour la mise en correspondance du référentiel de l'outil $R_o$ avec le référentiel du patient $R_p$ et le référentiel de l'image $R_i$ en fonction des informations provenant dudit capteur, des moyens pour déterminer les coordonnées de l'outil dans ledit référentiel fixe $R_c$ et des informations provenant de la base d'images, ledit calculateur délivrant un signal pour la visualisation de la position de l'outil dans le référentiel de l'image $R_i$ sur un écran de contrôle, et pour commander la position et les déplacements de l'outil en fonction de signaux de commande provenant d'un boîtier de contrôle.

Une telle installation permet d'exploiter une ou plusieurs images acquises préalablement à l'intervention, avant le transfert du patient en bloc chirurgical, et d'exploiter en temps réel les images en relation avec le déroulement de l'intervention chirurgicale.

Le référentiel fixe est un référentiel totalement indépendant est découplé tant du référentiel patient que du référentiel image, et du référentiel outil. Le référentiel fixe est un référentiel absolu et permanent. Il est par exemple lié à un élément de structure du bloc opératoire, par exemple le plafond, le sol ou un mur. Ce référentiel fixe est choisi de façon à garantir une référence permanente et stable dans laquelle les différentes matrices de transformations peuvent être calculées dans toutes les situations, en ne limitant ni les possibilités de déplacement du patient, ni les possibilités de déplacement de l'outil.

Selon une première variante, les capteurs sont constitués par au moins deux caméras d'acquisition solidaires du référentiel fixe $R_c$ et disposées de façon à ce que leur champs d'observation contiennent la zone d intervention chirurgicale.

Avantageusement, les moyens pour déterminer les coordonnées de l'outil dans ledit référentiel fixe $R_c$ sont constitués par au moins deux caméras d'acquisition solidaires du référentiel fixe $R_c$ et disposées de façon à ce que leur champs d'observation contiennent l'espace de mobilité de l'outil.

Selon un mode de réalisation préféré, l'installation comporte un trièdre géométriquement défini, présentant au moins quatre sources lumineuses ponctuelles non coplanaires solidaire du porte-outil, l'espace de mobilité dudit trièdre étant contenu dans le champs de vision des caméras d'acquisition.

Avantageusement l'installation comporte en outre un trièdre géométriquement défini, présentant au moins quatre sources lumineuses ponctuelles non coplanaires solidaire du patient, l'espace de mobilité dudit trièdre étant contenu dans le champs de vision des caméras d' acquisition.

L'invention sera mieux comprise à la lecture de la description qui suit, faisant référence aux dessins annexés où:

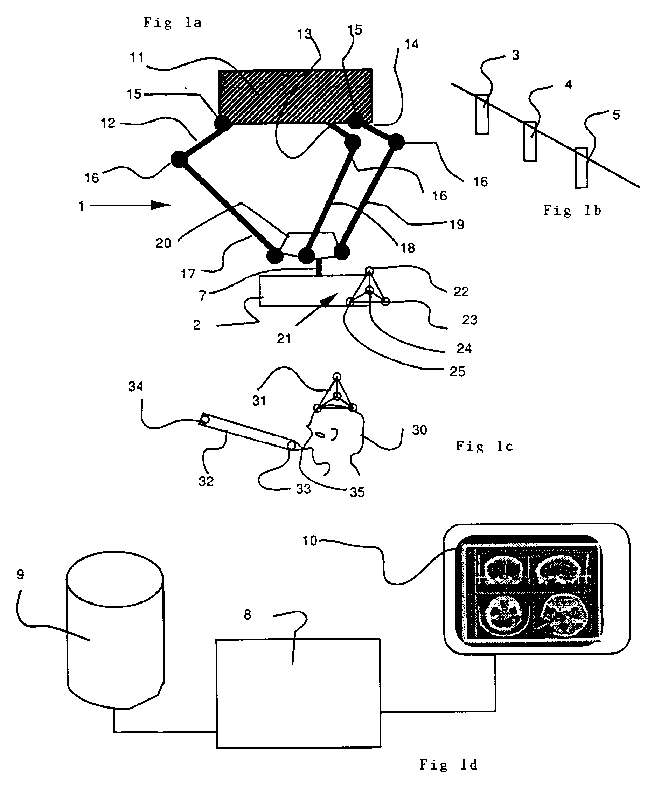

- La figure 1 représente une vue schématique de l'installation.

L'installation selon l'invention comporte:

- un support articulé (1) ;

- une platine porte-outil (2) ;
- un ensemble de trois caméras (3, 4, 5) ;
- des trièdres de référence (21, 31) ;
- un calculateur (8) ;
- un dispositif de stockage d'images numérisées (9) ;
- un écran de visualisation (10) .

Le support articulé (1) comporte une base (11) solidaire du référentiel fixe $R_c$ qui est par exemple le plafond de la salle d'opération.

Le support articulé (1) est constitué dans l'exemple décrit par un système de type "trois axes delta parallèle". Il comprend une première série de trois bras (12, 13, 14) reliés à la base (11) par des moteurs (15) commandés indépendamment. La première série de trois bras (12, 13, 14) est raccordée à une deuxième série de bras (17, 18, 19) par des rotules (16). Les extrémités des bras (17 à 19) sont solidaires d'une embase (20) par l'intermédiaire d'axes de rotation. Les bras sont écartés deux à deux de 120 degrés dans un plan parallèle à la base (11).

L'extrémité des bras (17 à 19) est relié à un mécanisme (20) comportant 3 axes de rotation perpendiculaires deux à deux, l'extrémité du dernier axe de rotation supportant une platine porte-outil (2) comportant des moyens d'accouplement d'un instrument chirurgical.

Cette embase comporte par ailleurs un trièdre (21) constitué par un assemblage de quatre points lumineux (22 à 25), par exemple des diodes électroluminescentes, dont la disposition géométrique est connue avec précision.

Le déplacement de ce trièdre (21) est acquis par l'ensemble de caméras (3, 4, 5) qui délivre un signal électrique permettant de calculer à tout moment la position du centre de gravité du trièdre (21) et son orientation, dans le référentiel fixe $R_c$, et donc de déterminer la matrice de passage entre le référentiel fixe $R_c$ et le référentiel du porte-outil $R_o$.

Les diodes électroluminescentes sont selon un mode de mise en oeuvre alimentées séquentiellement, la détection se faisant de manière synchrone.

Le patient (30) porte également un trièdre (31) grâce auquel l'ensemble de caméras (3, 4, 5) délivre un signal électrique permettant de calculer à tout moment la position du centre de gravité du trièdre (31) et son orientation, dans le référentiel fixe $R_c$, et donc de déterminer la matrice de passage entre le référentiel fixe $R_c$ et le référentiel du patient $R_p$.

On peut également réaliser le trièdre géométriquement défini sous forme d'implants mis en place sur le patient avant l'acquisition des images, et disposés en quatre points non alignés. Ces implants sont dans ce cas réalisés en un matériau permettant une détection par le ou les systèmes d'imagerie mis en oeuvre. Les implants sont par exemple réalisés en titane.

Le processus de mise en oeuvre de l'installation pour une intervention chirurgicale est le suivant:

Le patient, après une préparation, entre dans une première salle équipée de matériel d'acquisition des images. Dans cette salle, on procède de façon connue à l'instrumentation du patient, à l'acquisition des images brutes et à la vérification des images réalisées. Les images sont numérisées et stockées dans une base de données images. Ces images sont ensuite exploitées à partir d'une station de travail, en l'absence du patient, par calibration et segmentation des images, indexation des images et programmation éventuelle des trajectoires et des stratégies opératoires.

Le patient est ensuite transféré en salle d'opération.

Dans la salle d'opération, on procède successivement:

- à la préparation du patient
- à l'instrumentation du dispositif porte-outil ;
- à l'installation du patient, conservant l'instrumentation mise en place dans la phase d'acquisition d'images ;
- à l'instrumentation complémentaire du patient ;
- à la mise en correspondance des différents référentiels ;
- à l'intervention chirurgicale et à l'enregistrement des images opératoires.

L'instrumentation complémentaire sera seule visible en cours d'intervention chirurgicale, l'instrumentation initiale posée pendant la phase d'imagerie étant cachée sous les draps ou les champs.

Le patient est ensuite transféré hors de la salle opératoire, pendant que les images opératoires sont exploitées sur une station de travail.

Le processus d'acquisition des images par le système d'imagerie consiste plus particulièrement à :

- raser le patient, si on prévoit de lui instrumenter la tête ;
- anesthésier éventuellement le patient avant le transport en salle d'imagerie ;
- mettre en place le trièdre (15) ou les implants ;
- positionner le patient dans le système d'imagerie ;

- procéder à l'acquisition des images ;
- vérifier les images enregistrées dans la base d'images, notamment en ce qui concerne la visibilité des repères sur chacune des images enregistrées, de la définition, et des informations nécessaires à l'intervention chirurgicale ultérieure ;
- ressortir le patient.

Les images sont acquises par tout moyen d'imagerie connu, par exemple IRM, angiographie, radiographie, tomodensitométrie, etc.... Les images numérisées sont stockées dans une base de données éventuellement accessible par un réseau informatique à partir d'un site éloigné.

Les images ainsi enregistrées sont traitées en vue de procéder à :

- la calibration des images suivant les spécifications de l'imageur mis en oeuvre ;
- la segmentation des images en vue d'une exploitation 2D/3D ou 3D ;
- l'indexage éventuel des points de repères en vue de la mise en correspondance ;
- le repérage des points caractéristiques des images contenues dans la base de données images, en vue de l'exploitation pendant la phase opératoire, notamment par la recherche des cibles, des voies d'accès possibles et des trajectoires des instruments, et éventuellement par la simulation des différentes stratégies en 2D ou 3D, et une mémorisation des axes de progression ainsi expérimentés.

Après cette étape de traitement des images et d'exploitation virtuelle de la base de données images, le patient est transféré dans la salle opératoire.

Afin de permettre au chirurgien d'exploiter les informations préalablement acquises, il est nécessaire de connaître la position et l'orientation relative de l'axe de l'outil par rapport aux images, dans le repère intermédiaire correspondant à la zone d'intervention sur le patient.

A Cet effet, l'invention permet la mise en correspondance des images acquises et liées au patient, avec l'outil. Le repérage doit s'effectuer quelle que soit la position de l'outil et du patient.

Le trièdre (21) servant à repérer la position de l'outil est fixé de façon amovible ou non sur la base du porte-outil. Le moyen de fixation sera de préférence dépourvu d'articulation de façon à garantir une permanence de la position du trièdre (21) par rapport au support de l'outil. La solidarisation peut être réalisée par clipsage.

Le repérage du patient peut être réalisé de différentes façons: soit par pose d'un trièdre rigide normalisé, soit par pose d'implants non alignés, soit encore par désignation de points caractéristiques de la surface du patient, à proximité de la zone opératoire, avec un stylet de repérage.

Cette dernière solution consiste à mettre en oeuvre un pointeur (32) en forme de stylet, portant deux points de références détectables par le système de caméras, et permettant de désigner, et donc de mémoriser la position de différents points caractéristiques du patient, dont il est possible de suivre les déplacements par reconnaissance de forme. Ces zones caractéristiques sont par exemple le nez, les coins des yeux ou le menton.

Une telle sonde (32) comporte un corps en forme de stylet terminé par une zone de pointage (35), et comportant au moins deux points lumineux (33, 34) permettant de déterminer la position et l'orientation de la sonde (32) par analyse des signaux délivrés par les caméras (3, 4, 5).

La mise en concordance des référentiels sera exposée plus en détail dans ce qui suit.

Pour la bonne compréhension, on désignera par :

- $^aP$ un point défini dans le repère $R_a$ ;
- $^aT_b$ la matrice de transformation homogène (4 lignes, 4 colonnes) permettant d'exprimer dans le repère $R_a$ les coordonnées d'un point défini dans le repère $R_b$, par la relation $^aP = {^aT_b}\,{^bP}$.

Par ailleurs, les différents repères cités sont:

$R_0$ Repère de l'outil;
$R_i$ Repère de l'image ;
$R_c$ Repère des caméras ;
$R_{pr}$ Repère de la sonde ;
$R_{pg}$ Repère grossier du patient ;
$R_{pc}$ Repère corrigé du patient ;
$R_{mi}$ Repère géométrique défini par au moins 4 points non alignés (i variant de 1 à n) ;
$R_{m1}$ Repère géométrique lié à l'outil ;
$R_{m2}$ Repère géométrique lié au patient.

Par ailleurs, on notera $^{pr}S$ la surface définie par un ensemble de points $P_j$ acquis dans le repère sonde $R_{pr}$ et $^iS$ la surface définie par un ensemble de points $P_j$ acquis dans le repère image $R_i$.

Etape 1 : Mise en concordance entre le repère image et le repère patient

La première étape de la mise en concordance des référentiels consiste à calculer la matrice $^iT_{p/pc}$ de passage entre le repère image et le repère patient.

Selon un exemple de mise en oeuvre de l'installation, on utilise une sonde (32) afin de pointer des points remarquables connus dans le repère image $R_i$. Les coordonnées des extrémités de la sonde (32) sont connues par construction, et par traitement des informations délivrées par les caméras (3, 4, 5) détectant les points lumineux (33, 34) portés par la sonde.

On peut ainsi exprimer les coordonnées de l'extrémité (35) de la sonde (32) dans le repère de la caméra par la relation :

$$^cP_{\text{extrémité sonde}} = {^cT_{pr}} \, {^{pr}P_{\text{extrémité sonde}}}$$

et donc calculer la matrice de passage entre le référentiel de la caméra et le référentiel de la sonde.

On utilise par ailleurs des inserts ou un trièdre (31) comportant dans l'un ou l'autre des cas quatre points non alignés identifiables par les caméras (3 à 5), et définissant le repère $R_{pc}$ du patient.

Ces points $^iP_j$ sont connus dans le repère image $R_i$ et sont mesurés avec la sonde (32), dans le repère sonde $R_{pr}$ dans lequel leurs coordonnées sont $^{pr}P_j$. Lorsque l'extrémité de la sonde point sur l'un des points du trièdre (31) ou sur l'un des inserts, on a une relation d'identité entre les deux coordonnées :

$$^{pr}P_j = {^{pr}P_{\text{extrémité sonde}}}.$$

La transformation $^iT_{pr}$ est donc déterminée par une relation entre les points $^iP_j$ de la base de données images et les points $^{pc}P_j$ mesurés avec la sonde. On utilise le repère intermédiaire $R_{m2}$ fixe par principe d'utilisation, par rapport au repère $R_{pc}$ et on détermine la matrice de transformation $^iT_{m2}$. Cette matrice $^iT_{m2}$ est déterminée par une relation entre les points $^iP_j$ de la base de données images et les points $^{m2}p_j$ mesurés avec la sonde.

En effet, lorsque l'extrémité de la sonde (32) pointe sur un point $P_j$, la relation suivante est vérifiée:

$$^{m2}P_j = {^{m2}T_c(t)} \, {^cT_{pr}(t)} \, {^{pr}P_{\text{extrémité sonde}}}$$

et on détermine alors $^iT_{m2}$ par la méthode des moindres carrés:

$$\text{Min} \sum_{j=1}^{n} \|({^iP_j} - {^iT_{m2}} \, {^{m2}P_j})2\| \text{ avec } n \geq j \geq 4$$

Selon une variante de mise en oeuvre, on évite la pose d'un trièdre (31) ou la pose d'inserts, en utilisant une méthode de mise en correspondance surfacique.

On procède pour cela à deux étapes consécutives:

La première étape consiste à pointer 4 points remarquables sur le patient (par exemple le nez, les yeux etc...). On se retrouve alors dans une situation analogue à la variante précédente, car on dispose de points $P_j$ non coplanaires, dont les coordonnées $^iP_j$ sont connus dans le repère image $R_i$. La transformation $^iT_{pg}$ est déterminée par une relation entre les points $^iP_j$ de la base de données images et les points $^{pg}P_j$ mesurés avec la sonde (32).

On utilise comme précédemment le repère intermédiaire $R_{m2}$ fixe par rapport aux repères $R_{pg}$ et $R_{pc}$.

On obtient alors une transformation "grossière" $(^iT_{m2})_g$ qui permet d'obtenir une précision de l'ordre de quelques millimètres, insuffisante pour un usage clinique.

La deuxième étape consiste à définir un repère patient corrigé $R_{pc}$ en pointant une pluralité de points remarquables se trouvant à proximité de la zone d'intervention, à l'aide de la sonde (32).

Cette opération permet de mettre en correspondance deux surfaces:

- la surface réelle du patient, définie par l'acquisition faite avec la sonde $^{pr}S(^{pr}P_j)$ avec $n \geq j \geq 4$, la résolution étant d'autant meilleure que n est grand ;

- la surface $^iS$ liée à l'image du patient la plus proche de la surface réelle définie dans le repère image, et utilisant la transformation grossière $(^iT_{m2})_g$ en ne sélectionnant à cet effet qu'une partie de la banque de données images. $^{pr}S\{^{pr}P_j\}$ avec $n \geq j \geq 4$

On a alors la relation suivante:

$$^{m2}P_j = {}^{m2}T_c(t) \, {}^cT_{pr}(t) \, {}^{pr}P_{\text{extrémité sonde}}$$

avec $^{pr}P_{\text{extrémité sonde}} = {}^{pr}P_j$
et on détermine alors $^iT_{m2}$ par la méthode des moindres carrés :

$$\text{Min } \Sigma |(^iS\{P_j\} - {}^iT_{m2} \, {}^{m2}S\{^{m2}P_j\})^2| \text{ avec } n \geq j \geq 4$$

Etape 2 : Mise en concordance entre le repère outil et le repère fixe

L'étape suivante de la mise en concordance des référentiels consiste à calculer la matrice $^cT_o$ de passage entre le repère outil et le repère fixe.

- La transformation $^{m1}T_o$ donnant la relation entre le repère outil $R_o$ et le repère fixe $Rm_1$ est connu par construction.

Les coordonnées d'un point $^oP$ dans le repère $R_o$ peuvent être exprimées dans le repère $R_{m1}$ par la relation :

$$^{m1}P = {}^{m1}T_o \, {}^oP$$

- La transformation $^cT_{m1}$ donnant la relation entre le repère fixe $Rm_1$ et le repère $R_c$ est connu en temps réel par mesure infrarouge. Les coordonnées d'un point $^{m1}P$ dans le repère $R_{m1}$ peuvent être exprimées dans le repère $R_c$ par la relation :

$$^cP = {}^cT_{m1}(t) \, {}^{m1}P$$

Les coordonnées d'un point $^oP$ lié à l'outil peuvent donc être exprimés en temps réel dans le repère fixe de mesure $R_c$ par la relation :

$$^cP = {}^cT_{m1}(t) \, {}^{m1}T_o \, {}^oP$$

Le repère $R_o$ étant défini par le trièdre (21), on obtient ainsi la relation en temps réel entre le repère outil $R_o$ et le repère caméra $R_c$.

Résolution des équations permettant de calculer les matrices de transformation

Le repère fixe $R_{m1}$ est défini par au moins 4 points non coplanaires $^{m1}P_1$ à $^{m1}P_4$.
Les caméras (3 à 5) détectent ces quatre points dans le référentiel caméras, dans lequel leurs coordonnées sont $^cP_1$ à $^cP_4$.
On cherche la relation $^cT_{m1}$ telle que :

$$^cP_j = {}^cT_{m1} \, {}^{m1}P_j \text{ , où } j = 1 \text{ à } 4$$

Théoriquement, $||^cP_j - {}^cT_{m1} \, {}^{m1}P_j|| = 0$

On cherche donc $^cT_{m1}$ qui minimise les erreurs, d'où :

$$\text{Minimum} \left( \sum_{j=1}^{4} \| (^{c}P_{j} - {}^{c}T_{m1}\, {}^{m1}P_{j})^{2} \| \right)$$

Le minimum est déterminé par dérivation.

$^{c}T_{m1}$ est une matrice 4x4 homogène avec 12 éléments remarquables

$$^{C}T_{m1} = \begin{bmatrix} T_{11} & T_{12} & T_{13} & T_{14} \\ T_{11} & T_{22} & T_{23} & T_{24} \\ T_{31} & T_{32} & T_{33} & T_{34} \\ 0 & 0 & 0 & 1 \end{bmatrix}$$

On dérive la relation

$$S = \sum_{j=1}^{4} \| (^{c}P_{j} - {}^{c}T_{m1}\, {}^{m1}P_{j}) \|^{2}$$

$$\frac{\delta s}{\delta T(k,l)} = 0$$

et on obtient un système de 3x4 = 12 équations à 12 inconnues

pour k = 1 à 3 : k étant l'indice de ligne
pour 1 = 1 à 4, l étant l'indice de colonne

$$S = \sum_{j=1}^{4} \left[ \sum_{p=1}^{3} \left( {}^{c}P_{j(p)} - \sum_{q=1}^{4} {}^{c}T_{m1(p,q)}\, {}^{m1}P_{j(q)} \right)^{2} \right]$$

Comme

$$\frac{\delta s}{\delta T(k,l)} = 0,$$

on en déduit les relations suivantes :

$$\sum_{j=1}^{4} \left[ \sum_{p=1}^{3} \frac{\delta s}{\delta T(k,l)} [\,{}^{c}P_{j(p)} - \sum_{q=1}^{4} {}^{c}T_{m1(p,q)}\, {}^{m1}P_{j(q)}]^{2}) \right] = 0$$

et

$$\sum_{j=1}^{4} \left[ {}^{c}T_{m1(k,l)}\, {}^{m1}P_{j(l)} \left( {}^{c}P_{j(k)} - \sum_{q=1}^{4} {}^{c}T_{m1}(k,q)\, {}^{m1}P_{j(q)} \right) \right] = 0$$

Dans cette équation, on a:

$T_{k,l}$
$T_{k1}$
$T_{k2}$
$T_{k3}$
$T_{k4}$

Le système d'équation obtenu par

$$\frac{\delta s}{\delta T(k,l)} = 0 \text{ avec } k = 1 \text{ à } 3 \text{ et } l = 1 \text{ à } 4$$

se décompose en 3 sous-systèmes indépendants

$$\frac{\delta s}{\delta T(1,l)} = 0$$

$$\frac{\delta s}{\delta T(2,l)} = 0$$

$$\frac{\delta s}{\delta T(3,l)} = 0$$

La résolution de ce système d'équation est réalisée par un algorithme connu par le calculateur de l'installation selon l'invention, qui ne sera pas exposé plus en détail dans le cadre de la présente description, l'Homme du métier étant en mesure de mettre en oeuvre les solutions informatiques adaptées.

Etape 3 : Mise en concordance entre le repère image et le repère caméra.

L'étape suivante de la mise en concordance des référentiels consiste à calculer en temps réel la matrice $^{m2}T_i(t)$ de passage entre le repère $Rm_2$ lié au patient, avec le repère image $R_i$.

- La transformation $^cT_{pr}$ donnant la relation entre le repère $R_{pr}$ de la sonde (32) et le repère caméra $R_c$ est connu en temps réel par mesure infrarouge.

Les coordonnées d'un point $^{pr}P$ dans le repère $R_{pr}$ peuvent être exprimées dans le repère $R_c$ par la relation :

$$^cP = \ ^cT_{pr(t)} \ ^{pr}P.$$

- La transformation $^cT_{m2}$ donnant la relation entre le repère fixe $Rm_2$ et le repère $R_c$ est connu en temps réel par mesure infrarouge. Les coordonnées d'un point $^{m2}p$ dans le repère $R_{m2}$ peuvent être exprimées dans le repère $R_c$ par la relation :
  $^cP = \ ^cT_{m2}(t) \ ^{m2}P$ où $^cT_{m2}(t)$ est determinée de manière analogue à $^cT_{m1}(t)$.
- Les coordonnées de l'extrémité de la sonde (32) $^cP_{\text{extrémité sonde}}$ sont connues dans le repère $R_{pr}$ par construction.

Elles peuvent être exprimées par la relation:

$$^cP_{\text{extrémité sonde}} = \ ^cT_{pr}(t) \ ^{pr}P_{\text{extrémité sonde}}$$

Elles peuvent donc être exprimées dans le repère $R_{m2}$ par la relation :

$$^{m2}P_{\text{extrémité sonde}} = \ ^{m2}T_c \ ^cT_{pr}(t) \ ^{pr}P_{\text{extrémité sonde}}$$

<u>Etape 4 : Mise en concordance entre le repère image et le repère outil.</u>

L'étape finale de la mise en concordance consiste à déterminer la relation entre le repère $R_o$ repère image $R_i$.

On connaît pour cela :

- Etape 2 : la position de l'outil dans le repère des caméras par les transformation $^{m1}T_{o\_}$ (connue par construction) et $^cT_{m1}(t)$ (déterminée en temps réel par mesure infrarouge) ;
- Etape 3 : la corrélation entre le repère fixe $R_{m2}$ et le repère image $R_i$ par la transformation $^iT_{m2}$, déterminée lors de la mise en correspondance.
- la position du repère $R_{m2}$ par rapport au repère fixe $R_c$ par la transformation $^{m2}T_{c(t)}$ qui est l'inverse de $^cT_{m2(t)}$, déterminée en temps réel par mesure infrarouge.

On obtient donc la transformation

$$^iT_{o(t)} = \, ^iT_{m2} \, ^{m2}T_{c(t)} \, ^cT_{m1(t)} \, ^{m1}T_0$$

permettant d'afficher en temps réel la coupe correspondant au point d'intérêt.

On obtient également la transformation $^oT_{i(t)}$, inverse de $^iT_{o(t)\_}$ permettant d'asservir l'outil en temps réel par rapport à une cible définie dans la base de données images.

L'invention est décrite dans ce qui précède à titre d'exemple non limitatif. Il est bien entendu que l'Homme de Métier sera à même de proposer diverses variantes sans pour autant sortir du cadre de l'invention.

**Revendications**

1. Installation pour opération de microchirurgie assistée par ordinateur, du type comportant un support d'outils articulé dont l'une des extrémités est solidaire d'un premier référentiel $R_c$, ainsi qu'une base de données Image dans laquelle sont enregistrées les images provenant d'un système d'imagerie dans le référentiel de l'image $R_i$ ledit premier référentiel $R_c$ est un référentiel fixe indépendant du patient et du référentiel image et en ce que l'installation comporte des moyens pour déterminer les coordonnées de l'outil dans ledit référentiel fixe $R_c$, caractérisée en ce que l'installation comporte au moins un capteur bidimensionnel solidaire du premier référentiel délivrant un signal électrique fonction de la position du référentiel du parient $R_p$ dans le premier référentiel $R_c$, et un calculateur pour la mise en correspondance du référentiel de l'outil $R_o$ avec le référentiel du patient $R_p$ et le référentiel de l'image $R_i$ en fonction des informations provenant du capteur bidimensionnel, le calculateur délivrant un siqnal pour la visualisation de la position de l'outil dans le référentiel de l'image $R_i$ sur un écran de contrôle, et pour commander la position et les déplacements de l'outil en fonction de signaux de commande provenant d'un boîtier de contrôle.

2. Installation pour opération de microchirurgie assistée par ordinateur selon la revendication 1 caractérisée en ce que le capteur bidimensionnel est constitué par au moins deux caméras d'acquisition solidaires du référentiel fixe $R_c$ et disposées de façon à ce que leur champs d'observation contiennent la zone d'intervention chirurgicale.

3. Installation pour opération de microchirurgie assistée par ordinateur selon la revendication 1 ou selon la revendication 2 caractérisée en ce que les moyens pour déterminer les coordonnées de l'outil dans ledit référentiel fixe $R_c$ sont constitués par au moins deux caméras d'acquisition solidaires du référentiel fixe $R_c$ et disposées de façon à ce que leur champs d'observation contiennent l'espace de mobilité de l'outil.

4. Installation pour opération de microchirurgie assistée par ordinateur selon l'une quelconque des revendications précédentes caractérisée en ce qu'elle comporte un trièdre géométriquement défini, présentant au moins quatre sources lumineuses ponctuelles non coplanaires solidaire de l'outil, l'espace de mobilité dudit trièdre étant contenu dans les champs de vision des caméras d'acquisition.

5. Installation pour opération de microchirurgie assistée par ordinateur selon l'une quelconque dus revendications précédentes caractérisée en ce qu'elle comporte un trièdre géométriquement défini, présentant au moins quatre sources lumineuses ponctuelles non coplanaires solidaire de du patient. l'espace de mobilité dudit trièdre étant contenu dans le champs de vision des caméras d'acquisition, pendant la totalité de la phase opératoire.

6. Installation pour opération de microchirurgie assistée par ordinateur selon la revendication 1 caractérisée en ce qu'elle comporte en outre une sonde (32) présentant une extrémité de pointage (35) et au moins deux points lumineux (33, 34) dont les positions par rapport à l'extrémité de pointage (35) sont déterminée géométriquement.

7. Procédé de visualisation de la position d'un outil de microchurgie par rapport à une image préenregistrée caractérisé en ce qu'il comporte les étapes suivantes :

- détermination à l'aide d'une caméra liée au repère $R_c$ de la position de l'outil dans le repère $R_c$ par les transformation $^{m1}T_{o\_}$ connue par construction et $^cT_{m1(t)}$, déterminée en temps réel par mesure optique

  où $^{m1}T_o$ désigne la matrice de transformation donnant la relation entre le repère outil $R_o$ et le repère $R_{m1}$ connu par construction
  et $^cT_{m1(t)}$ désigne la matrice de transformation donnant la relation entre le repère fixe $R_{m1}$ et le repère $R_c$ à l'instant t ;

- détermination de la matrice $^iT_{m2}$ de passage entre un repère $R_{m2}$ lié au patient et le repère image Rj ;
- détermination de la position du repère $R_{m2}$ par rapport au repère $R_c$ par la transformation $^{m2}T_{c(t)}$ déterminée en temps réel par mesure optique ;
- calcul de la transformation

$$^iT_{o(t)} = {}^iT_{m2}\, {}^{m2}T_{c(t)}\, {}^cT_{m1(t)}\, {}^{m1}T_0$$

  permettant d'afficher on temps réel la coupe correspondant au point d'intérêt.

8. Procédé d'asservissement d'un outil de microchurgie par rapport à une base de données images caractérise on ce qu'il comporte les étapes suivantes :

- détermination à l'aide d'une caméra de la position de l'outil dans le repère $R_c$ de la caméra par les transformations $^{m1}T_{o\_}$ connue par construction donnant la relation entre le repère $R_o$ et le repère fixe $R_{m1}$ et $^cT_{m1(t)}$ donnant la relation entre le repère fixe $Rm_1$ et le repère $R_c$, déterminée en temps réel par mesure optique ;
- détermination de la matrice $^iT_{m2}$ de passage entre le repère fixe $R_{m2}$ lié au patient. et le repère image $R_i$;
- détermination de la position du repère $R_{m2}$ par rapport au repère fixe $R_c$ par la transformation $^{m2}T_{c(t)}$ déterminée en temps réel par mesure optique ;
- calcul de la transformation

$$^iT_{o(t)} = {}^iT_{m2}\, {}^{m2}T_{c(t)}\, {}^cT_{m1(t)}\, {}^{m1}T_0$$

- calcul de la transformation $^oT_{i(t)}$, inverse de $^iT_{o(t)\_}$ permettant d'asservir l'outil en temps réel par rapport à une cible définie dans la base de données images.

**Claims**

1. computer-aided microsurgical operation installation comprising an articulated tool holder, one of the ends of which is linked to a first reference point $R_c$, and an Image database in which the images from an imaging system are recorded in the image reference point $R_i$, said first reference point $R_c$ being a fixed reference point independent of the patient and the image reference point and the installation comprising equipment to determine the co-ordinates of the tool in said fixed reference point $R_c$, characterised in that the installation comprises at least one two-dimensional sensor linked to the first reference point emitting an electric signal according to the position of the patient reference point $R_p$ in the first reference point $R_c$ and a computer to determine the correspondence of the tool reference point $R_o$ with the patient reference point $R_p$ and the image reference point $R_i$ according to the data sent by the two-dimensional sensor, with the computer emitting a signal for the display of the position of the tool in the image reference point $R_i$ on a monitor, and to control the position and movements of the tool according to control signals from a control unit.

2. Computer-aided microsurgical operation installation according to claim 1 characterised in that the two-dimensional sensor is composed of at least two acquisition cameras linked to the fixed reference point $R_c$ and arranged such that their observation fields contain the surgical operative area.

**3.** Computer-aided microsurgical operation installation according to claim 1 or according to claim 2 characterised in that the equipment to determine the co-ordinates of the tool in said fixed reference point $R_c$ is composed of at least two acquisition cameras linked to the fixed reference point $R_c$ and arranged such that their observation fields contain the tool mobility field.

**4.** Computer-aided microsurgical operation installation according to any of the above claims characterised in that it comprises a geometrically defined trihedron with at least four non-coplanar individual light sources linked to the tool, the mobility field of said trihedron being contained in the fields of vision of the acquisition cameras.

**5.** Computer-aided microsurgical operation installation according to any of the above claims characterised in that it comprises a geometrically defined trihedron with at least four non-coplanar individual light sources linked to the patient, the mobility field of said trihedron being contained in the fields of vision of the acquisition cameras, throughout the entire operative phase.

**6.** Computer-aided microsurgical operation installation according to claim 1 characterised in that it also comprises a probe (32) with one pointing end (35) and at least two light points (33, 34) for which the positions with reference to the pointing end (35) are geometrically defined.

**7.** Process to display the position of a microsurgical tool with reference to a pre-recorded image characterised in that it comprises the following stages :

- determination using a camera linked to the reference point $R_c$ of the position of the tool in the reference point $R_c$ by the transformations $^{m1}T_o$ known by design and $^cT_{m1(t)}$, determined in real time by optical measurement ;

  where $^{m1}T_o$ refers to the transformation matrix giving the relationship between the tool reference point $R_o$ and the reference point $R_{m1}$ known by design,
  and $^cT_{m1(t)}$ refers to the transformation matrix giving the relationship between the fixed reference point $R_{m1}$ and the reference point $R_c$ at the time t ;

- determination of the transition matrix $^iT_{m2}$ between a reference point $R_{m2}$ linked to the patient and the image reference point $R_i$ ;
- determination of the position of the reference point $R_{m2}$ with reference to the reference point $R_c$ by the transformation $^{m2}T_{c(t)}$ determined in real time by optical measurement ;
- calculation of the transformation

$$^iT_{o(t)} = {}^iT_{m2}\ {}^{m2}T_{c(t)}\ {}^{cT}m_{1(t)}\ {}^{M1}T_o$$

  making it possible to display the section corresponding to the point of interest in real time.

**8.** Control process for a microsurgical tool with reference to an image database characterised in that it comprises the following stages :

- determination using a camera of the position of the tool in the camera reference point $R_c$ by the transformations $^{m1}T_o$, known by design, giving the relationship between the reference point $R_c$ and the fixed reference point $R_{m1}$, and $^cT_{m1(t)}$, giving the relationship between the fixed reference point $R_{m1}$ and the reference point $R_c$, determined in real time by optical measurement;
- determination of the transition matrix $^iT_{m2}$ between the reference point $R_{m2}$ linked to the patient and the image reference point $R_i$ ;
- determination of the position of the reference point $R_{m2}$ with reference to the fixed reference point $R_c$, by the transformation $^{m2}T_{c(t)}$ determined in real time by optical measurement ;
- calculation of the transformation

$$^iT_{o(t)} = {}^iT_{m2}\ {}^{m2}T_{c(t)}\ {}^{cT}m_{1(t)}\ {}^{m1}T_o$$

- calculation of the transformation $^oT_{i(t)}$, the inverse of $^iT_{o(t)}$, making it possible to control the tool in real time with reference to a target defined in the image database.

**Patentansprüche**

1. Anlage für computergestützte Mikrochirurgie-Operationen, des Typs mit einem gelenkigen Werkzeugträger, dessen eines Ende mit einem ersten Bezugssystem $R_c$ verbunden ist, sowie mit einer Bilddatenbasis, in der die von einem Bildersystem im Bezugssystem des Bildes $R_1$ kommenden Bilder aufgezeichnet sind, wobei das erste Bezugssystem $R_c$ ein festes, vom Patienten und vom Bild-Bezugssystem unabhängiges Bezugssystem ist und die Anlage Mittel umfasst, um die Koordinaten des Werkzeugs im besagten festen Bezugssystem $R_c$ zu bestimmen, dadurch gekennzeichnet, dass die Anlage mindestens einen zweidimensionalen Geber umfasst, der mit dem ersten Bezugssystem verbunden ist und ein elektrisches Signal entsprechend der Position des Bezugssystems des Patienten $R_p$ im ersten Bezugssystem $R_c$ abgibt, und einen Rechner für die Abstimmung des Bezugssystems des Werkzeugs $R_o$ mit dem Bezugssystem des Patienten $R_p$ und dem Bezugssystem des Bildes $R_i$ entsprechend den vom zweidimensionalen Geber kommenden Informationen, wobei der Rechner ein Signal abgibt für die Visualisierung der Position des Werkzeugs im Bezugssystem des Bildes $R_i$ auf einem Kontrollbildschirm, und zum Steuern der Position und der Verschiebungen des Werkzeugs entsprechend der von einem Kontrollkasten kommenden Steuersignale.

2. Anlage für computergestützte Mikrochirurgie-Operationen nach Anspruch 1, dadurch gekennzeichnet, dass der zweidimensionale Geber aus mindestens zwei mit dem festen Bezugssystem $R_c$ verbundenen Erfassungskameras besteht, die so angeordnet sind, dass ihre Beobachtungsfelder die Zone des chirurgischen Eingriffs umfassen.

3. Anlage für computergestützte Mikrochirurgie-Operationen nach Anspruch 1 oder nach Anspruch 2, dadurch gekennzeichnet, dass die Mittel zur Bestimmung der Koordinaten des Werkzeug im besagten festen Bezugssystem $R_c$ aus mindestens zwei mit dem festen Bezugssystem $R_c$ verbundenen Erfassungskameras besteht, die so angeordnet sind, dass ihre Beobachtungsfelder den Bewegungsraum des Werkzeugs umfassen.

4. Anlage für computergesteuerte Mikrochirurgie-Operationen nach einem der vorstehend genannten Ansprüche, dadurch gekennzeichnet, dass sie einen geometrisch definierten Trieder umfasst, der mindestens vier nicht koplanare Punktlichtquellen aufweist und mit dem Werkzeug verbunden ist, wobei der Bewegungsraum des besagten Trieders in den Sichtfeldern der Erfassungskameras enthalten ist.

5. Anlage für computergesteuerte Mikrochirurgie-Operationen nach einem der vorstehend genannten Ansprüche, dadurch gekennzeichnet, dass sie einen geometrisch definierten Trieder umfasst, der mindestens vier nicht koplanare Punktlichtquellen aufweist und mit dem Patienten verbunden sind, wobei der Bewegungsraum des besagten Trieders während der gesamten Operationsphase in den Sichtfeldern der Erfassungskameras enthalten ist.

6. Anlage für computergesteuerte Mikrochirurgie-Operationen nach Anspruch 1, dadurch gekennzeichnet, dass sie weiters eine Sonde (32) aufweist, die ein Ausrichtungsende (35) und mindestens zwei Lichtpunkte (33, 34) umfasst, deren Positionen in Bezug auf das Ausrichtungsende (35) geometrisch vorgegeben sind.

7. Visualisierungsverfahren der Position eines Mikrochirurgie-Werkzeugs in Bezug auf ein voraufgezeichnetes Bild, dadurch gekennzeichnet, dass es folgende Etappen umfasst :

- Bestimmung mittels einer mit der Marke $R_c$ in Verbindung stehenden Kamera der Werkzeugposition in der Marke $R_c$ durch Umwandlung von $^{m1}T_o$, bekannt durch Konstruktion, und von $^{c}T_{m1(t)}$, bestimmt in Echtzeit durch optische Messung,

  wo $^{m1}T_o$ die Umwandlungsmatrize bezeichnet, die die Beziehung zwischen der Werkzeugmarke $R_o$ und der Marke $R_{m1}$, bekannt durch Konstruktion, angibt,
  und wo $^{c}T_{m1(t)}$ die Umwandlungsmatrize bezeichnet, die die Beziehung zwischen der festen Marke $R_{m1}$ und der Marke $R_c$ zum Zeitpunkt t angibt ;

- Bestimmung der Matrize $^{i}T_{m2}$ des Übergangs zwischen einer mit dem Patienten verbundenen Marke $R_{m2}$ und der Bildmarke $R_i$ ;
- Bestimmung der Position der Marke $R_{m2}$ in Bezug auf die Marke $R_c$ durch Umwandlung von $^{m2}T_{c(t)}$, bestimmt in Echtzeit durch optische Messung ;
- Berechung der Umwandlung

$$^iT_{o(t)} = \ ^iT_{m2} \ ^{m2}T_{c(t)} \ ^cT_{m1(t)} \ ^{m1}T_o$$

womit der dem Interessenpunkt entsprechende Schnitt in Echtzeit angezeigt werden kann.

8. Steuerverfahren eines Mikrochirurgie-Werkzeugs in Bezug auf eine Bilddatenbasis, dadurch gekennzeichnet, dass es folgende Etappen umfasst :

- Bestimmung mittels einer Kamera der Werkzeugposition in der Marke $M_c$ der Kamera durch die Umwandlungen von $^{m1}T_o$, bekannt durch Konstruktion, der die Beziehung zwischen der Marke $R_o$ und der festen Marke $R_{m1}$ angibt, und $^cT_{m1(t)}$, der die Beziehung zwischen der festen Marke $R_{m1}$ und der Marke $R_c$ angibt und per optischer Messung in Echtzeit bestimmt wird ;
- Bestimmung der Matrize $^iT_{m2}$ des Übergangs zwischen der mit dem Patienten verbundenen Marke $R_{m2}$ und der Bildmarke $R_i$ ;
- Bestimmung der Position der Marke $R_{m2}$ in Bezug auf die feste Marke $R_c$ durch Umwandlung von $^{m2}T_{c(t)}$, bestimmt in Echtzeit durch optische Messung ;
- Berechung der Umwandlung

$$^iT_{o(t)} = \ ^iT_{m2} \ ^{m2}T_{c(t)} \ ^cT_{m1(t)} \ ^{m1}T_o$$

- Berechnung der Umwandlung $^cT_{i(t)}$ umgekehrt zu $^iT_{o(t)}$, womit das Werkzeug in Echtzeit in Bezug auf ein in der Bilddatenbasis definiertes Ziel gesteuert werden kann.

Fig 1a

Fig 1b

Fig 1c

Fig 1d